# FASCICULE DE BREVET EUROPEEN

(11) **EP 1 948 721 B1**
(45) Date de publication et mention de la délivrance du brevet: **20.07.2016**
(21) Numéro de dépôt: 06820276.1
(22) Date de dépôt: 10.10.2006
(51) Int. Cl.: C08J 9/08, A61L 27/18, A61L 27/60

(54) **MATRICE POREUSE BIOCOMPATIBLE ET BIODEGRADABLE NOTAMMENT UTILE POUR LA RECONSTRUCTION TISSULAIRE**
BIOKOMPATIBLE UND BIOLOGISCH ABBAUBARE PORÖSE MATRIX, DIE INSBESONDERE FÜR DIE GEWEBEREKONSTRUKTION GEEIGNET IST
BIOCOMPATIBLE AND BIODEGRADABLE POROUS MATRIX IN PARTICULAR USEFUL FOR TISSUE RECONSTRUCTION

(30) Priorité: 11.10.2005 FR 0553088
(43) Date de publication de la demande: 30.07.2008
(73) Titulaire: Centre National de la Recherche Scientifique, 75794 Paris Cedex 16 (FR); Université Montpellier 1, 34000 Montpellier (FR)
(72) Inventeur: CASELLAS, Daniel, F-34080 Montpellier (FR); GARREAU, Henri, F-34270 Saint Mathieu De Treviers (FR); GARRIC, Xavier, F-34250 Pavalas-Les-Flots (FR); MOLES, Jean-Pierre, F-34660 Cournonsec (FR); VERT, Michel, F-34170 Castelnau-Le-Lez (FR)
(74) Mandataire: Paris, Fabienne
(86) Numéro de dépôt international: PCT/FR2006/051014
(87) Numéro de publication internationale: WO 2007/042728

(56) Documents cités:
- WO-A-97/19973
- US-B1- 6 562 374
- PORJAZOSKA ALEKSANDRA ET AL: "Synthesis of an ABA triblock copolymer of poly(DL-lactide) and poly(ethylene glycol) and blends with poly(epsilon-caprolactone) as a promising material for biomedical application" ACS SYMPOSIUM SERIES AMER CHEMICAL SOC, 1155 SIXTEENTH ST NW, WASHINGTON, DC 20036 USA SERIES : ACS SYMPOSIUM SERIES (ISSN 0097-6156(PRINT)), 12 septembre 2005 (2005-09-12), pages 119-135, XP009072051 & SYMPOSIUM ON NEW POLYMERIC MATERIALS; CAPRI, ITALY; OCTOBER 22 -25, 2003 ISSN: 0-8412-3928-2(H)
- WAKE M CONLEY ET AL: "Fabrication of pliable biodegradable polymer foams to engineer soft tissues" CELL TRANSPLANTATION, vol. 5, no. 4, 1996, pages 465-473, XP002398017 ISSN: 0963-6897
- HODGES D E ET AL: "Surgical adhesives for laser-assisted wound closure." JOURNAL OF BIOMEDICAL OPTICS. OCT 2001, vol. 6, no. 4, octobre 2001 (2001-10), pages 427-431, XP002398018 ISSN: 1083-3668
- CERRAI P ET AL: "BLOCK COPOLYMERS OF L-LACTIDE AND POLY(ETHYLENE GLYCOL) FOR BIOMEDICAL APPLICATIONS" JOURNAL OF MATERIALS SCIENCE. MATERIALS IN MEDICINE, CHAPMAN AND HALL, LONDON, GB, vol. 5, janvier 1994 (1994-01), pages 308-313, XP000606965 ISSN: 0957-4530 cité dans la demande
- CHEN W ET AL: "SYNTHESIS AND PROPERTIES OF POLY(L-LACTIDE)-POLY(ETHYLENE GLYCOL) MULTIBLOCK COPOLYMERS BY COUPLING TRIBLOCK COPOLYMERS" POLYMERS FOR ADVANCED TECHNOLOGIES, WILEY & SONS, BOGNOR REGIS, GB, vol. 14, no. 3-5, mars 2003 (2003-03), pages 245-253, XP001170478 ISSN: 1042-7147
- LIN HONG-RU ET AL: "Preparation of macroporous biodegradable PLGA scaffolds for cell attachment with the use of mixed salts as porogen additives" JOURNAL OF BIOMEDICAL MATERIALS RESEARCH, vol. 63, no. 3, 2002, pages 271-279, XP002398019 ISSN: 0021-9304 cité dans la demande

## Description

La présente invention se rapporte au domaine de la reconstruction des tissus de revêtement après perte de substance ou à celui des pansements bioactifs.

La présente invention concerne plus particulièrement une nouvelle matrice poreuse, biocompatible et biodégradable, utile pour la préparation d'échafaudages de tissu(s) conjonctif(s), ainsi que lesdits échafaudages. L'invention concerne en outre un substitut mésenchymateux comprenant ladite matrice poreuse ou ledit échafaudage de tissus conjonctif(s) associé à des cellules endothéliales et/ou fibroblastes et un substitut de tissu(s) de revêtement, leurs procédés de préparation ainsi que leurs utilisations.

D'une façon générale, dans le domaine de la reconstruction tissulaire avec perte de substance, les plus grandes avancées ont été effectuées en relation avec la reconstruction de l'os, du cartilage et de tissu(s) de revêtement, dont plus particulièrement de l'épiderme.

La reconstruction de l'épiderme est ainsi par exemple mise en oeuvre pour traiter de larges pertes de substance telles qu'observées chez les grands brûlés, et les pertes de substances locales comme dans les plaies chroniques et les ulcères. Généralement, la reconstruction de téguments, et notamment cutanés implique également la mise en oeuvre de pansements bioactifs dont le but est de favoriser la cicatrisation.

Concernant les substituts cutanés, la première des solutions développées après la seconde guerre mondiale consistait à greffer des tissus hétérogéniques. Des xénogreffes de cochons, puis des allogreffes de cadavres ont permis dans un premier temps de maintenir les malades en vie. Mais devant le nombre de rejets importants et l'avènement des connaissances en immunologie, les chercheurs ont commencé à s'orienter vers l'utilisation de tissus autologues dont la première technique fut l'autogreffe. Cette technique est désormais largement utilisée dans les services de chirurgie réparatrice pour traiter les ulcères cutanés et les brûlures du 2^{éme} et 3^{ème} degré.

L'autogreffe consiste à prélever des fragments de peau saine de nature dermo-épidermique pour les déposer sur des plaies préparées par les chirurgiens (lits de greffe). Cette technique d'autogreffe est très intéressante car la prise de greffe est rapide. Elle est également utilisée sous forme de pastilles pour traiter les ulcères cutanés. Mais cette technique de l'autogreffe est limitée car, en fonction de l'étendue de la plaie, il ne reste pas toujours suffisamment de peau saine à prélever même si on sait accroître dans certaines proportions la surface des autogreffes.

La reconstruction épidermique est une technique qui a été développée ultérieurement à l'autogreffe.

Le premier feuillet épidermique cultivé *in vitro* fut obtenu par Rheinwald et Green en 1975 (Cell 6: 331-343. Serial cultivation of strains of human keratinocytes: the formation of keratinizing colonies from single cells). Ce type de feuillet épidermique autologue (Epicel^{®}) est utilisé dans la majorité des services de grands brûlés et permet de traiter des malades dont le pronostic vital est engagé. Cette technique consiste à faire croître des kératinocytes autologues, prélevés chez le malade, en présence d'une couche nourricière de fibroblastes irradiés.

L'inconvénient de cette technique se situe au niveau du décollement du feuillet de son support de culture. En effet, il est nécessaire d'utiliser un traitement enzymatique à l'aide d'une protéinase, et en particulier la dispase, qui est responsable de la contraction et de l'altération du feuillet. Ces modifications jouent un rôle néfaste dans la prise de greffe.

Ces techniques et ces avancées représentent et représenteront un important progrès en thérapeutique, en particulier pour les brûlés, mais la cicatrice obtenue à partir de ces feuillets épidermiques, reposant directement sur le fascia, est imparfaite. En effet, l'épiderme est atrophique et on observe une fragilité durable de la peau greffée qui est donc de qualité insuffisante. L'absence de derme s'avère donc extrêmement préjudiciable pour la reconstruction cutanée et pour les propriétés mécaniques de cette nouvelle peau.

Pour pallier cette fragilité de la peau due à l'absence de derme sur le lit de greffe, il est apparu que la solution était de fabriquer un derme équivalent pour assurer le support mécanique de l'épiderme tout en permettant, immédiatement après la détersion de la plaie, de limiter les pertes volémiques et les risques d'infection. Dans cette optique, trois dermes équivalents ont été développés (Integra^{®}, Dermagraft^{®} et Transcyte^{®}) :
- Intégra^{®} (Integra LifeSciences Technology). Ce système de recouvrement de la plaie est composé de deux couches acellulaires : une première couche superficielle, mimant l'épiderme, est constituée d'une membrane en silicone et une seconde couche plus profonde, mimant le derme, est constituée de collagène de tendon de boeuf et de glycosaminoglycanes de requin. Ce substitut dermique forme une matrice poreuse dermique qui est destinée à être colonisée par les fibroblastes des tissus environnants. Après colonisation de cette matrice (21 jours), la couche mince de silicone est enlevée puis remplacée par un épiderme de culture.
- Dermagraft^{®} (Smith & Nephew) Il s'agit d'une résille résorbable en vicryl (Polyglactin 910) contenant des fibroblastes allogéniques. Ce substitut est vendu congelé, utilisable sur une plaie à n'importe quel moment. Il n'est pas rejeté malgré la présence de fibroblastes allogéniques. Ce derme équivalent est particulièrement utilisé dans le traitement des ulcères cutanés. La présence d'un échafaudage dégradable hydrolytiquement suppose que seule la présence d'eau est nécessaire à sa dégradation et ceci n'implique donc pas l'intervention de l'organisme. Toutefois, cette dégradation peut être à l'origine de la libération de produits de dégradation plus ou moins néfastes à la prolifération kératinocytaire, notamment l'acide glycolique.
- Transcyte^{®} (Advanced Tissue Science) est un substitut dermiques formé d'un réseau de nylon (polyamide) sur lequel ont été cultivés des fibroblastes dermiques allogéniques. Ce réseau est enduit d'une couche de collagène porcin de type I puis recouvert d'une membrane en silicone jouant le rôle d'épiderme synthétique.

Ces trois substituts dermiques améliorent les résultats de prise de greffe et de cicatrisation. Il faut toutefois noter que la présence de fibroblastes allogéniques contenus dans Dermagraft^{®} et Transcyte^{®} accélère la réapparition d'un néoderme fonctionnel. Il est alors possible de retrouver des propriétés mécaniques proches du *in vivo* (en raison d'une néosynthèse rapide de fibres élastiques).

Ces techniques favorisent donc la prise de greffe mais il est nécessaire de greffer un épiderme de culture sur ces dermes équivalents. L'épiderme et le derme étant cultivés séparément, la jonction dermo-épidermique est absente et ne se forme qu'au cours de la cicatrisation. Il en résulte une moins bonne résistance à l'arrachement de l'épiderme et un défaut de cohésion de ce substitut de tissu(s) de revêtement.

Le produit Apligraf^{®} (Novartis) se présente quant à lui comme un complexe dermo-épidermique qui possède un derme de collagène bovin de type I comportant des fibroblastes allogéniques, un épiderme cultivé *in vitro* et une jonction dermo-épidermique. Ce substitut de tissu(s) de revêtement peut être assimilé à la peau dans sa composition en deux couches reliées par la jonction dermo-épidermique mais présente deux inconvénients majeurs qui ont été les motifs du refus de commercialisation en Europe :
- La présence de collagène étranger induit une réaction de l'hôte dont le but est de détruire ces fibres de collagène pour ensuite en synthétiser de nouvelles qui seront orientées dans le sens de tension de la peau,
- La présence de cellules allogéniques (fibroblastes et kératinocytes) qui risquent de provoquer, particulièrement pour les kératinocytes, une réaction de rejet de l'organisme receveur. Toutefois, l'utilisation de ces cellules allogéniques permet de réduire au maximum les délais de culture et de pouvoir greffer le substitut cutané sur un lit de greffe préparé.

On remarque, d'après la revue des techniques rappelées ci-dessus, que les matériaux proposés jusqu'à ce jour peuvent se diviser en trois groupes : les matériaux d'origine naturelle, les semi-artificiels et les artificiels. Les matériaux d'origine naturelle comprennent les matrices acellulaires telles que celles composées de collagène, d'alginate, de glycosaminoglycanes, d'hydroxyapatite ou de fibrine. Les matériaux artificiels incluent en grande majorité les matériaux dérivés des polymères artificiels que sont les poly(acide lactique), poly(acide glycolique), polyéthylène glycol, polyphosphazènes et de nombreux hydrogels. Les matériaux semi-artificiels combinent les matériaux d'origine naturelle et artificielle.

Enfin, parmi les dernières solutions proposées mettant en oeuvre des matériaux artificiels pour la reconstruction de téguments, et notamment cutanés après perte de substance, on peut citer un substitut dermique composé d'un échafaudage de copolymère poly(téréphtalate de butylène)-co-poly(téréphtalate d'éthylène glycol), décrit dans El-Ghalbzouri A, Lamme EN, van Blitterswijk C, Koopman J, Ponec M. « The use of PEGT/PBT as dermal scaffold for skin tissue engineering. » Biomaterials 2004 ; 25(15) : 2987-96.

Parallèlement au domaine de la reconstruction tissulaire avec large perte de substance, il existe également un large domaine d'application pour la reconstruction tissulaire avec perte de substance locale ou cicatrisation. D'ailleurs, la plupart des substituts de tissu(s) de revêtement décrits précédemment ont également été proposés comme pansements bioactifs destinés à favoriser transitoirement la cicatrisation. Ces aides à la cicatrisation sont particulièrement importantes lors du traitement des plaies chroniques tels que les ulcères cutanés.

Il existe par conséquent un besoin de trouver un substitut de tissu(s) de revêtement, notamment d'épiderme destiné à remplacer les autogreffes et les allogreffes jusqu'à présent utilisées en chirurgie réparatrice pour les affections cutanées dont le pronostic vital est engagé et ne présentant pas les inconvénients détaillés ci-dessus, ce substitut pouvant également trouver une application comme pansement bioactif destiné à favoriser la cicatrisation.

Il existe de plus un besoin de trouver un échafaudage de tissu(s) conjonctif(s) à base d'un matériau artificiel de qualité accrue en terme de prise de greffe et présentant une bonne aptitude à la biodégradation et des risques de reconnaissance immunitaire réduits.

La présente invention a plus précisément pour objet une matrice poreuse biocompatible et biodégradable, caractérisée en ce qu'elle comprend un copolymère séquencé tribloc répondant à la formule (I) :

X-G-Y (I)

dans laquelle :
G est un bloc linéaire hydrophile, non hydroxylé, contenant p motifs répétitifs, p étant un nombre pouvant varier de 150 à 700,
X et Y, identiques ou différents, représentent respectivement un bloc polyester linéaire hydrophobe, X et Y pouvant chacun respectivement contenir n et m motifs répétitifs, n et m étant chacun un nombre pouvant varier de 170 à 3500, et en ce qu'elle possède des pores de diamètre variant de 20 à 500 µm.

Selon un mode de réalisation avantageux, la matrice poreuse selon l'invention présente un caractère hydrophile.

L'invention concerne en outre un procédé de préparation d'une matrice telle que définie ci-dessus, caractérisé en ce qu'il comprend au moins une étape de préparation d'un copolymère tel que défini précédemment, suivi d'une étape de formation de pores au sein du copolymère ainsi préparé.

L'invention a par ailleurs pour objet un échafaudage de tissu(s) conjonctif(s), comprenant au moins une matrice telle que définie ci-dessus, en association avec au moins un agent de comblement biocompatible et dégradable.

Elle concerne de plus un substitut mésenchymateux, utile pour la reconstruction de tissu(s) conjonctif(s) et/ou pour la cicatrisation, comprenant une matrice poreuse ou un échafaudage de tissu(s) conjonctif(s) tels que définis précédemment, en association avec des cellules endothéliales et/ou fibroblastes.

Elle a également pour objet un procédé de préparation dudit substitut mésenchymateux tel que défini ci-dessus, comprenant une étape de mise en contact d'une matrice poreuse ou d'un échafaudage de tissu(s) conjonctif(s) tels que décrits précédemment, avec des cellules endothéliales et/ou fibroblastes puis une étape de prolifération cellulaire.

Elle a aussi pour objet un substitut de tissu(s) de revêtement caractérisé en ce qu'il comprend au moins une matrice poreuse, un échafaudage de tissu(s) conjonctif(s) et/ou un substitut mésenchymateux tels que définis précédemment en association avec des cellules épithéliales.

L'invention a également pour objet un procédé de préparation dudit substitut de tissu(s) de revêtement, caractérisé en ce qu'il comprend une étape de mise en contact d'une matrice poreuse, d'un échafaudage de tissu(s) conjonctif(s) et/ou d'un substitut mésenchymateux tels que définis précédemment avec des cellules épithéliales, puis une étape de prolifération cellulaire.

Enfin, la présente invention concerne l'utilisation de la matrice poreuse selon l'invention pour l'élaboration d'un échafaudage de tissu(s) conjonctif(s) tel que défini précédemment ou équivalent.

Elle concerne également l'utilisation d'une matrice poreuse ou d'un échafaudage de tissu(s) conjonctif(s) selon l'invention pour l'obtention d'un substitut mésenchymateux tel que défini précédemment ou équivalent.

Elle concerne aussi l'utilisation d'un substitut mésenchymateux selon l'invention pour l'obtention d'un substitut de tissu(s) de revêtement tel que défini précédemment.

Elle a également pour objet une utilisation de la matrice poreuse, de l'échafaudage de tissu(s) conjonctif(s), du substitut mésenchymateux ou du substitut de tissu(s) de revêtement tels que définis ci-dessus pour la préparation d'un matériau destiné à la réparation de téguments, notamment cutanés, et/ou pour la préparation d'un pansement bioactif destiné à favoriser la cicatrisation.

Les matrices poreuses, échafaudages de tissu(s) conjonctif(s), substituts mésenchymateux et/ou substituts de tissu(s) de revêtement selon l'invention peuvent également être utilisés à des fins de diagnostics. Plus précisément, les matériaux selon l'invention cités ci-dessus peuvent être mis en oeuvre à des fins d'évaluation par exemple de la toxicité, l'activité, la tolérance et/ou l'impact de composés, de compositions pharmaceutiques, voire de techniques de traitement destiné(e)s à être administré(e)s et/ou à être mis(es) en contact avec un tissu de revêtement tel que la peau par exemple.

La présente invention a ainsi également pour objet, l'utilisation d'une matrice poreuse, d'un échafaudage de tissu(s) conjonctif(s), d'un substitut mésenchymateux et/ou d'un substitut de tissu(s) de revêtement à des fins de réalisation d'un test de diagnostic *in vitro.*

Dans le cadre de la présente invention, on entend par :
- « bloc », une partie d'une macromolécule comprenant plusieurs unités constitutives identiques ou différentes et qui possède au moins une particularité de constitution ou de configuration permettant de la distinguer de ses parties adjacentes,
- « matériau biocompatible », un matériau qui est capable de remplir sa fonction sans effet néfaste sur l'environnement biologique dans lequel il est introduit. Dans les cas d'une reconstruction tissulaire, sa fonction de support cellulaire et de substitut d'organes implique que la biocompatibilité englobe également la notion de cytocompatibilité ou aptitude du matériau en tant que support de culture. Le deuxième axe principal de la biocompatibilité est l'innocuité du matériau vis-à-vis de l'organisme,
- « matériau biodégradable », un matériau qui peut être altéré au contact des cellules vivantes de telle façon que son intégrité soit altérée au niveau moléculaire par un processus lié à l'activité biologique. En particulier, la notion de biodégradabilité implique une perte de propriété, éventuellement une disparition du site d'implantation mais pas nécessairement une élimination de l'organisme,
- « matériau biorésorbable », un matériau qui se dégrade pour l'essentiel et pour lequel on a la preuve que les produits de dégradation sont intégrés en biomasse et/ou éliminés de l'organisme par métabolisation ou filtration rénale,
- « tégument », un tissu périphérique qui constitue pour le corps de l'animal une paroi continue, à laquelle on peut reconnaître un rôle général de soutien et de protection vis-à-vis du milieu extérieur. Par exemple, dans le cas particulier de la peau, le terme « tégument » ne se réfère pas seulement aux tissus et formations proprement épidermiques, mais également aux tissus sous-jacents compris dans la dénomination vague de « derme »,
- « pansement bioactif », un dispositif médical pouvant être déposé ou implanté de façon temporaire ou définitive, sur un tissu pour en stimuler la reconstruction, ledit pansement pouvant, selon une alternative, être actif au contact d'un organisme vivant ou d'un extrait d'organisme vivant,
- « échafaudage de tissu(s) conjonetif(s) », un matériau acellulaire en association avec au moins un agent de comblement apte à servir de structure à une culture cellulaire destinée à former un substitut mésenchymateux,
- « substitut mésenchymateux », le matériau résultant de la culture des cellules endothéliales et/ou fibroblastes au contact d'un échafaudage de tissu(s) conjonctif(s), d'une matrice poreuse ou équivalent,
- « substitut de tissu(s) de revêtement », le matériau combinant une barrière synthétique (ex. silicone) ou biologique (ex. épithélium de revêtement) et un substitut mésenchymateux ou un échafaudage de tissu(s) conjonctif(s),
- « épithélium de revêtement », un tissu formé de cellules jointives,.
   juxtaposées, solidaires les unes des autres par des systèmes de jonction et séparées du tissu adjacent par une lame basale, qui recouvre l'intérieur du corps et certaines cavités de l'organisme. Les cavités de l'organisme plus particulièrement considérées selon l'invention, sont un prolongement de l'extérieur telles les voies aériennes, le tube digestif, les voies urinaires et les voies génitales.

A titre d'exemple non limitatif d'épithéliums de revêtement considérés selon l'invention, on peut notamment citer l'épiderme, les muqueuses ou les canaux des glandes exocrines.

Les épithéliums de revêtement peuvent être monocouches (une seule couche de cellule), stratifiés ou pseudo stratifiés.

Les termes « compris entre ... et ... » et « varier de ... à ... » signifient que les bornes sont également décrites.

### MATRICE POREUSE BIOCOMPATIBLE ET BIODEGRADABLE

La matrice comprend un copolymère séquencé tribloc répondant à la formule (I):

X-G-Y (I)

dans laquelle:
G est un bloc linéaire hydrophile, non hydroxylé, contenant p motifs répétitifs, p étant un nombre pouvant varier de 150 à 700,
X et Y, identiques ou différents, représentent respectivement un bloc polyester linéaire hydrophobe, X et Y pouvant chacun respectivement contenir n et m motifs répétitifs, n et m chacun étant un nombre pouvant varier de 170 à 3500, en particulier de 200 à 3000 et notamment de 300 à 2500.

Des copolymères apparentés aux copolymères définis ci-dessus sont connus en tant que tels. Ils sont notamment connus pour leur utilisation pour former les hydrogels décrits dans le brevet EP 863 933 B1.

Selon une variante privilégiée de l'invention, n et m peuvent varier indépendamment l'un de l'autre de 200 à 3000 et de façon encore plus préférée de 300 à 2500.
p varie avantageusement de 180 à 650 et de façon encore plus préférée de 200 à 600.

Selon une autre variante privilégiée de l'invention, le rapport (m+n)/p est compris entre 0,48 et 47.

Ce rapport est compris avantageusement entre 0,60 et 33 et plus particulièrement entre 1 et 25, par exemple entre 1 et 15.

Le copolymère selon l'invention est avantageusement hydrophile.

Les blocs que représentent X et Y, dans la formule (I), sont notamment des polyesters aliphatiques.

On sait que les polyesters aliphatiques peuvent être obtenus :
a) par polycondensation d'un hydroxyacide sur lui-même, ou par polycondensation de plusieurs hydroxyacides,
b) par polymérisation par ouverture de cycle lactone,
c) ou encore par polycondensation de diacides et de diols.

Les polyesters aliphatiques utilisables pour la synthèse d'une matrice selon l'invention, comprennent, mais ne sont pas limités, aux homopolymères et copolymères de lactide (incluant l'acide lactique, D-,L- et meso-lactide) ; glycolide (incluant l'acide glycolique) ; ε-caprolactone ; p-dioxanone (1,4-dioxan-2-one) ; carbonate de triméthylène (1,3-dioxan-2-one); les dérivés alkyles du carbonate de triméthylène ; δ-valérolactone ; β-butyrolactone ; γ-butyrolactone ; ε-décalactone ; hydroxybutyrate : hydroxyvalérate ; 1,4-dioxépan-2-one (incluant ses dimères 1,5,8,12-tétraoxacyclotétradécane-7,14-dione) ; 1,5-dioxépan-2-one ; 6,6-diméthyl-1,4-dioxan-2-one ; 2,5-dikétomorpholine ; pivalolactone ; α, α diéthylpropriolactone ; carbonate d'éthylène ; oxalate d'éthylène ; 3-méthyl-1,4-dioxane-2,5-dione ; 3,3-diethyl-1,4-dioxan-2,5-dione ; 6,6-diméthyl-dioxépan-2-one ; 6,8-dioxabicycloctane-7-one et leurs mélanges de polymères.

Les polymères aliphatiques utilisés dans la présente invention peuvent être des homopolymères ou des copolymères (statistiques ou blocs, en peigne ou alternés).

Plus précisément, le polyester aliphatique peut notamment être choisi parmi (i) un homopolymère qui dérive des monomères choisis parmi l'acide lactique, les monoesters d'acide malique, les lactides, le glycolide, la para-dioxanone, l'ε-caprolactone, (ii) un copolymère qui dérive des monomères précités, ou (iii) un polymère obtenu par polycondensation de diacides et de diols.

Parmi les polyesters dérivés d'hydroxyacides, on peut citer notamment ceux qui dérivent des monomères choisis parmi l'acide lactique, l'acide glycolique, les monoesters d'acide malique (par exemple des monoesters d'alkyle ou d'aralkyle), ou encore des monoesters résultant de la monoestérification de l'acide malique par un principe actif hydroxylé, notamment un principe actif hydrophobe (voir par exemple les brevets US 4 320 753 et 4 265 247), les lactides, le glycolide ou la para-dioxanone. Les blocs représentés par X et Y peuvent également être des copolymères formés par lesdits monomères entre eux.

Parmi les polymères dérivés de diacides et de diols, on peut citer par exemple le poly(succinate d'éthylène glycol).

Dans le cadre de la présente invention, le terme « lactide » comprend le L-lactide, le D-lactide, le meso-lactide, et leurs mélanges.

Le bloc polymère représenté par G dans la formule (I) est un polymère hydrophile qui peut être choisi par exemple parmi les suivants : poly(éthylèneglycol), poly(vinylpyrrolidone), poly(vinyl alcohol), polyoxazoline et copolymères analogues.

Selon une variante privilégiée de l'invention, le bloc polymère G est un bloc poly(éthylène glycol) ou PEG de formule H(OCH₂CH₂)ₚOH, où p varie de 200 à 600.

Avantageusement, le copolymère présente une faible longueur de chaîne au niveau du bloc polymère G, de manière à conférer une dégradation adaptée à son utilisation à titre d'échafaudage de tissu(s) conjonctif(s), retenue pour la matrice correspondante.

Dans le cadre de la présente invention, les copolymères séquencés triblocs de formule (I) peuvent présenter une masse molaire moyenne en nombre comprise entre 31 000 et 550 000, de préférence entre 37 500 et 475 000, et de façon encore plus préférée entre 45 000 et 400 000, par exemple entre 65 000 et 400 000.

On peut effectuer la polymérisation conduisant à la formation des chaînons X et Y en présence du polymère G préformé et ayant des extrémités convenablement fonctionnalisées. Par exemple, on a déjà décrit l'obtention de copolymères séquencés à base de poly(hydroxyacides) et de polyéthylèneglycol ; voir notamment « Block copolymers of L-lactide and poly(ethylene glycol) for biomedical applications » P. Cerrai et al., Journal of Materials Science : Materials in Medicine 5 (1994) 308-313 et le document EP 295 055. Les produits de départ sont d'une part le lactide et d'autre part le polyéthylène glycol. On opère de préférence par polymérisation en masse en présence d'un catalyseur qui peut être un métal, un composé organo-métallique ou un acide de Lewis. Parmi les catalyseurs utilisés, on peut citer la poudre de zinc, l'hydrure de calcium, l'hydrure de sodium, l'octanoate d'étain, le lactate de zinc, etc. La longueur des chaînes poly(hydroxyacide) dépend essentiellement du rapport molaire (motifs lactiques)/(PEG) dans le mélange initial. La longueur des chaînes poly(hydroxyacide) croît également avec la durée de la réaction qui peut aller par exemple de quelques minutes à quelques jours.

En utilisant des blocs polymères de type G aux extrémités fonctionnalisées, portant par exemple des groupes terminaux hydroxyle, acide carboxylique, amino, thiol, etc., il est possible d'obtenir des copolymères séquencés, répondant à la formule (I) ci-dessus, dans lesquels la jonction entre X et G et entre Y et G se fait par l'intermédiaire d'un groupement hydrolysable tel que ester, amide, uréthanne, thioester, etc.

Les copolymères de formule (I) peuvent également être préparés à partir de blocs polymères préformés, en utilisant un polymère de type G dont les deux extrémités sont, de façon connue, convenablement fonctionnalisées, et des polymères de type X et Y ayant une seule extrémité fonctionnalisée de façon à pouvoir réagir avec une extrémité fonctionnalisée de G, avec établissement d'une liaison covalente.

La matrice selon la présente invention est avantageusement poreuse. Cette porosité joue un double rôle. Ainsi, elle permet l'hébergement de cellules lors de la phase de préparation du substitut mésenchymateux selon la présente invention puis sa vascularisation une fois en place sur le tissu hôte. Elle confère par ailleurs des propriétés mécaniques, à savoir une souplesse améliorée, permettant d'obtenir une bonne compatibilité avec le milieu environnant. On note par ailleurs que le copolymère ne doit pas être altéré par la réaction de formation des pores et enfin les pores doivent être interconnectées de façon à autoriser la colonisation cellulaire et le passage de fluides nutritifs.

On peut utiliser une variante du procédé de formation de pores décrit dans Hong-Ru Lin et al, « Preparation of macroporous biodegradable PLGA scaffolds for cell attachment with the use of mixed salts as porogen additives », J Biomed Mater Res 2002;63(3):271-9. Ainsi, ce procédé décrit des mélanges chlorure de sodium/hydrogéno carbonate d'ammonium à température supérieure à 60 °C.

On peut avantageusement utiliser dans le cadre de la présente invention un procédé comportant au moins une étape de formation de pores consistant à utiliser à titre d'agent générateur de porosité des particules comprenant de l'hydrogénocarbonate d'ammonium (NH₄HCO₃), préférentiellement à titre d'unique constituant.

La génération de ces pores est régie par un phénomène assimilable à un phénomène de percolation. Il relève des compétences de l'homme de métier de mettre en oeuvre les techniques connues pour obtenir des matrices poreuses selon la présente invention.

Selon un mode de réalisation avantageux de l'invention, on utilise une teneur en un tel agent générateur de porosité telle que le rapport massique entre la teneur en agent générateur de porosité et la teneur en copolymère de formule (I) varie de 1/1 à 50/1, de préférence de 5/1 à 30/1.

En particulier, lorsque l'agent générateur de porosité est l'hydrogénocarbonate d'ammonium, et le copolymère de formule (I) est un copolymère tribloc polylactide-polyéthylèneglycol-polylactide, et notamment le copolymère tel qu'illustré à l'exemple 1, ce rapport massique varie avantageusement de 1/1 à 50/1 et de préférence de 5/1 à 30/1.

Ces particules peuvent être complètement extraites de la matrice polymère grâce à un procédé de lavage en deux étapes à 70 °C par immersion dans l'eau. Les pores ainsi obtenus peuvent présenter un diamètre compris entre 20 µm et 500 µm.

D'autres techniques classiques de formation de pores peuvent alternativement être mises en oeuvre mais celles-ci doivent conduire à une porosité ouverte compatible avec l'invasion cellulaire.

Selon une variante privilégiée de l'invention, le diamètre des pores de la matrice varie de 20 à 500 µm de préférence de 150 à 500 µm. Les quantifications de la porosité, du diamètre des pores, et de l'interconnexion peuvent être obtenues par examen au Microscope Electronique à Balayage, préférentiellement Environnemental (MEBE). Il est également possible de déterminer le diamètre et le nombre de pores par microscopie optique ou encore de démontrer la connectivité par passage d'une solution aqueuse à travers la matrice poreuse sèche à un débit d'au moins 200 µL par minute par centimètre carré.

Bien entendu, la densité et le diamètre des pores sont liés à la nature de l'agent générateur de porosité utilisé et au protocole expérimental appliqué.

Le caractère biocompatible de la matrice poreuse selon la présente invention se décline en deux axes, comme l'a souligné la définition donnée ci-dessus.

D'une part, l'innocuité de la matrice et de ses produits de dégradation vis-à-vis de l'organisme est recherchée. Quatre critères cumulatifs permettent de vérifier que ce premier axe est respecté, à savoir l'absence de cytotoxicité, l'absence d'immunogénicité, l'absence de thrombogénicité et l'absence de mutagénicité. Ainsi, dans le cadre de la présente invention, la matrice poreuse répond avantageusement favorablement à l'ensemble des critères exposés ci-dessus.

D'autre part, au niveau cellulaire, la matrice présente de plus avantageusement une bonne cytocompatibilité.

En particulier, cette cytocompatibilité peut être mise en évidence par une étude d'adhésion des cellules constitutives des téguments (principalement les fibroblastes et les cellules épithéliales), une étude de prolifération, une étude de la conservation du phénotype et une étude de vascularisation.

Outre ce caractère biocompatible, d'autres propriétés sont recherchées pour permettre l'utilisation de la matrice selon la présente invention à des fins de réalisation d'échafaudages de tissu(s) conjonctif(s). Ainsi, la matrice présente de préférence une malléabilité et une résistance à la traction, qui s'approchent le plus possible des caractéristiques mécaniques des tissus conjonctifs.

La matrice est avantageusement biodégradable, voire biorésorbable.

Enfin, la matrice présente avantageusement la propriété d'être stérilisable et donc compatible avec une greffe dans des conditions d'asepsie optimales tout en n'affectant pas les propriétés de la matrice.

La matrice poreuse, objet de l'invention, peut en tant que telle être utilisée à titre de pansement bioactif.

Selon un mode de réalisation préféré de l'invention, le copolymère conforme à l'invention présente une masse molaire moyenne en nombre comprise entre 65 000 et 400 000 et présente un rapport (m+n)/p compris entre 1 et 15.

Selon un mode de réalisation tout particulièrement préféré, le copolymère conforme à la présente invention présente une masse molaire moyenne comprise entre 80 000 et 375 000 et présente un rapport ((n+m)/p) compris entre 1,8 et 10,9.

Une matrice poreuse comprenant un copolymère selon ce mode de réalisation particulier présente en effet de nombreux avantages.

Ainsi, une telle matrice poreuse présente des propriétés mécaniques particulièrement adaptées à ses applications détaillées ci-après telle que son utilisation pour la préparation d'échafaudages de tissu(s) conjonctif(s), de substitut mésenchymateux, de substitut de tissu(s) de revêtement et/ou de matériau pour la réparation de téguments et/ou à titre de pansement bioactif pour favoriser la cicatrisation. En effet, elle présente alors une malléabilité et une résistance à la traction appropriées les rendant tout particulièrement aptes à la manipulation chirurgicale. Notamment, une telle matrice poreuse est apte à la suture.

Par ailleurs, la dégradation hydrolytique est également adaptée dans le sens où la matrice poreuse conserve une intégrité adéquate lors de son application en tant que telle ou sous l'une des formes d'application listées ci-dessus.

De plus, une telle matrice poreuse, prise en tant que telle ou sous l'une des formes d'application listées ci-dessus présente l'avantage de résister aux exsudats.

Enfin, ce mode de réalisation préféré est particulièrement avantageux concernant la prolifération cellulaire. Ainsi, une telle matrice poreuse, prise en tant que telle ou sous l'une des formes d'application listées ci-dessus, présente une prolifération cellulaire optimisée grâce à la balance hydrophile-hydrophobe particulière découlant de ce mode de réalisation.

### ECHAFAUDAGE DE TISSU(S) CONJONCTIF(S)

L'échafaudage de tissu(s) conjonctif(s) selon la présente invention est caractérisé en ce qu'il comprend au moins une matrice poreuse biocompatible et biodégradable telle que définie précédemment.

Selon un mode dé réalisation particulier de l'invention, l'échafaudage peut être imprégné d'un agent de comblement biocompatible et dégradable de sorte à favoriser l'ancrage des cellules dans ledit échafaudage. L'agent de comblement peut notamment être choisi parmi les matériaux biologiques ou non contenant du collagène et/ou de la fibrine.

A titre d'agent de comblement, on peut citer l'acide hyaluronique hydrophobisé, le plasma riche en fibrine coagulée, préférentiellement autologue, un gel de collagène ou un gel d'alginate.

Selon un mode de réalisation privilégié de l'invention, la teneur en agent de comblement biocompatible et dégradable et plus particulièrement en collagène, est généralement inférieure à 10 %, et notamment comprise entre 0,05 et 7 %, voire entre 0,1 et 3 % en poids sec par rapport au poids total exprimé en matière sèche de l'échafaudage de tissu(s) conjonctif(s).

Tout autre composé pouvant participer à la reconstruction tissulaire, à la cicatrisation, à l'asepsie, à la lutte contre la douleur et l'inflammation peut également être inclus dans l'échafaudage. A titre d'exemple, on peut citer des facteurs de croissance, des antibiotiques, des sels d'argent, des antifongiques, des anti-inflammatoires et des antitumoraux.

L'échafaudage de tissu(s) conjonctif(s) peut présenter toutes les formes de sorte à s'adapter à la géométrie et aux dimensions des lésions de tissu(s) de revêtement, et notamment cutanées à traiter.

L'échafaudage de tissu(s) conjonctif(s) peut par ailleurs présenter une épaisseur variant de 500 µm à 5 mm selon les applications requises.

Enfin, l'échafaudage de tissu(s) conjonctif(s) peut comprendre une matrice poreuse telle que définie précédemment et une autre type de matrice sous forme de couches successives pour autant que les propriétés de l'échafaudage ne s'en trouvent pas affectées, notamment en terme de biodégradabilité, de biocompatibilité, de propriétés mécaniques, etc.

Ces matériaux sont particulièrement intéressants pour la reconstitution de tégument. Les inventeurs ont en effet constaté que ces matériaux intervenaient activement dans le processus de régénération cellulaire épithéliale. Ils ont par exemple un effet bénéfique de cicatrisation vis-à-vis des tissus ayant subi un dommage. En tant que tels, ils agissent comme un pansement bioactif.

Selon une variante de réalisation, la matière poreuse, l'échafaudage de tissu(s) conjonctif(s), le substitut mésenchymateux et/ou le substitut de tissu(s) de revêtement peuvent être revêtus en surface d'un film organique ou inorganique et notamment de type silicone. Ce matériau de revêtement ainsi revêtu peut être utilisé tel quel ou débarrassé de ce film amovible avant usage. Dans le cas d'un usage tel quel on privilégie bien entendu une disposition en surface du film organique ou inorganique au moment de l'application.

Par exemple l'échafaudage de tissu(s) conjonctif(s) peut comprendre un film de polymère type silicone à titre de film protecteur.

### SUBSTITUT MESENCHYMATEUX

La présente invention a également pour objet un substitut mésenchymateux caractérisé en ce qu'il comprend une matrice poreuse telle que définie précédemment ou un échafaudage de tissu(s) conjonctif(s) conforme à l'invention en association avec des cellules endothéliales et/ou fibroblastes.

Le substitut mésenchymateux de la présente invention peut être utilisé à titre de pansement bioactif ou à titre de substitut de tissu(s) de revêtement. Dans cette alternative, on obtient ainsi un substitut de tissu(s) de revêtement directement *in vivo* sans soumettre le substitut mésenchymateux à une prolifération de cellules épithéliales *in vitro,* comme cela est exposé ci-après. En d'autres termes, les cellules épithéliales de l'organisme hôte prolifèrent *in site,* formant ainsi un substitut de tissu(s) de revêtement *in situ.*

Il peut être obtenu par culture de cellules endothéliales et/ou fibroblastes au contact d'une matrice poreuse ou d'un échafaudage de tissu(s) conjonctif(s) tels que précédemment définis.

Plus particulièrement, lorsque l'on vise la fabrication d'un substitut mésenchymateux, les pores de la matrice selon la présente invention peuvent avantageusement être chargés de l'agent de comblement tel que défini ci-dessus, et/ou de cellules endothéliales et/ou fibroblastes, de préférence de fibroblastes. Le substitut mésenchymateux ainsi obtenu peut alors être implanté directement ou bien subir au préalable une étape de prolifération cellulaire *in vitro,* pour donner un pansement bioactif et/ou un substitut de tissu(s) de revêtement.

L'étape de prolifération fibroblastique a avantageusement lieu dans des conditions de culture avec milieu supplémenté pour une durée comprise par exemple entre trois et quinze jours.

L'exemple 4.1 illustre cet aspect de l'invention.

### SUBSTITUT DE TISSUS DE REVETEMENT

Le substitut de tissu(s) de revêtement selon la présente invention, est caractérisé en ce qu'il comprend au moins un substitut mésenchymateux en association avec des cellules épithéliales.

Les cellules épithéliales peuvent être plus particulièrement choisies parmi les cellules épithéliales de l'appareil gastro-intestinal (cavité buccale, notamment les cellules gingivales, oesophage, estomac, intestin grêle, gros intestin et rectum), des bronches, de l'appareil gastro-urinaire (vessie) et de l'appareil génital (vagin) et les kératinocytes dans le cas de cellules épithéliales de l'épiderme.

Le substitut de tissu(s) de revêtement de la présente invention peut être utilisé à titre de substitut de tissu(s) de revêtement biodégradable et/ou à titre de pansement bioactif.

Il peut être obtenu par culture de cellules épithéliales telles que les kératinocytes au contact d'un substitut mésenchymateux ou d'un échafaudage de tissu(s) conjonctif(s) tel que précédemment définis. Le substitut de tissu(s) de revêtement biodégradable ainsi obtenu peut être implanté directement ou bien subir une étape de prolifération cellulaire *in vitro.* L'étape de prolifération kératinocytaire peut avoir lieu dans des conditions de culture, dans un milieu avec ou sans sérum, par exemple pour une durée comprise entre trois et trente jours.

L'exemple 5 illustre cet aspect de l'invention.

Les quatre produits précédemment décrits peuvent être utilisés à titre de pansements bioactifs. Ils peuvent notamment être déposés ou implantés au niveau de tous les épithéliums décrits précédemment et plus particulièrement de la peau, mais encore au niveau des gencives.

Les inventeurs ont par ailleurs démontré que ces quatre produits présentent des propriétés pro-angiogéniques *in vivo.*

Ainsi, la présente invention se rapporte tout particulièrement à une matrice poreuse, un échafaudage, un substitut mésenchymateux, un substitut de tissu(s) de revêtement, un matériau pour la réparation de téguments et à un pansement bioactif conformes à la présente invention, dotés de propriétés pro-angiogéniques.

Les affections et plus particulièrement endommagements de tissu(s) de revêtement, et notamment cutanées susceptibles d'être traitées par l'implantation de chacun des quatre produits précédemment décrits à savoir la matière poreuse, l'échafaudage de tissu(s) conjonctif(s), le substitut mésenchymateux, le substitut de tissu(s) de revêtement selon la présente invention sont en particulier les naevi, les ulcères cutanés et les brûlures.

L'origine du terme « naevus » désigne une malformation de tissu(s) de revêtement, et notamment cutanée ayant cliniquement l'aspect d'une tumeur bénigne et correspondant à un « hamartome ». Actuellement, ce terme est plus restrictif et utilisé au sens de naevus pigmentaire ou naevus noevocellulaire.

Ces tumeurs sont composées de cellules næviques (ou nævocytes) disposées en thèques. Selon la localisation des thèques næviques dans les plans tégumentaires, et notamment cutanés, on distingue le naevus nævocellulaire dermique, le nævus nævocellulaire jonctionnel, le nævus nævocellulaire mixte ou composé et le nævus bleu.

L'ulcère cutané se définit quant à lui comme une perte de substance cutanée chronique sans tendance spontanée à la cicatrisation. Il ne s'agit pas d'une maladie en soi mais de la complication d'une maladie vasculaire sous-jacente souvent ancienne ou grave qui règle le pronostic et la conduite thérapeutique. L'ulcère de jambe, très fréquent, est invalidant et à l'origine de très nombreuses hospitalisations.

Enfin, la brûlure est une lésion du revêtement tégumentaire, et notamment cutané due à un transfert d'énergie entre une source de chaleur et le tégument. Les brûlures les plus fréquentes sont thermiques. Elles peuvent être également d'origine électrique, chimique ou par rayonnement ionisant.

La présente invention est illustrée par les exemples qui suivent, sans toutefois en limiter la portée.

### Exemple 1 : Préparation d'une matrice poreuse conforme à la présente invention

### 1.1. Synthèse d'un copolymère PLA₅₀-PEG-PLA₅₀

Le D,L-Lactide (Sigma-Aldrich) a été purifié par recristallisation. Un PEG 20 000-dihydroxyle (Fluka) a été séché sous vide avant utilisation. L'amorceur utilisé, le dilactate de zinc (Sigma-Aldrich) a également été séché sous vide.

Le copolymère tribloc (composé de deux segments de PLA₅₀ et un segment de PEG20000) a été obtenu par polymérisation par ouverture de D,L-Lactide en présence de PEG20000 et de dilactate de zinc selon le protocole suivant.

Les réactifs (D,L-Lactide 32,7 g ; PEG20000 5 g ; dilactate de zinc 7 mg) sont séchés sous vide puis placés dans un ballon à col long (ballon de polymérisation) ensuite raccordé au montage.

Un robinet à trois voies permet d'établir les communications mélange réactionnel/vide ou mélange réactionnel/Argon. Le mélange est porté à fusion sous vide. Le long col du ballon est refroidi (papier humide ou réfrigérant) pour récupérer, par recondensation, les éventuels produits de sublimation/distillation.

Le dégazage consiste en une série de purges Argon/vide, le mélange étant alternativement liquide ou solide (par refroidissement). Ce dégazage permet d'éliminer toute trace d'oxygène qui est un inhibiteur de la polymérisation.

Le ballon est ensuite scellé sous vide dynamique au chalumeau. La polymérisation est réalisée dans un four muni d'un système d'agitation par rotation.

Après polymérisation (15 jours), les copolymères sont dissous dans l'acétone, puis précipités au méthanol. Ils sont ensuite séchés sous vide jusqu'à masse constante.

Le copolymère synthétisé a été caractérisé par spectroscopie Infra-Rouge et spectroscopie RMN ¹H. La masse molaire moyenne en nombre est de 85 000 g/mol évaluée d'après l'analyse par RMN du proton.

Dans cet exemple, n = 451 ; m = 451 ; p = 454 et le rapport (m+n)/p est égal à 1,98.

Parallèlement à la synthèse du copolymère, un PLA₅₀ a été synthétisé pour servir de contrôle pour l'ensemble des investigations. Ce PLA₅₀ a été caractérisé par Chromatographie d'Exclusion Stérique : la masse molaire moyenne en nombre est de 50 000 g/mol et l'indice de polymolécularité (Ip) de 1,4.

### 1.2. Formation de matrices poreuses

La technique de solubilisation de sels développée par Hong-Ru Lin *et al.,* citée plus haut, a été utilisée pour fabriquer une matrice poreuse en PLA₅₀-PEG-PLA₅₀.

Des particules d'hydrogénocarbonate d'ammonium (NH₄HCO₃, Sigma-Aldrich) ont été tamisées (<250 µm) puis séchées sous vide. Ces particules ont ensuite été mélangées avec une solution de polymère selon l'exemple 1.1. dans l'acétone (1 mg/ml). Le rapport en masse NH₄HCO₃/PLA₅₀-PEG-PLA₅₀ était de 9/1. Le mélange a enfin été déposé dans une boite de pétri en verre à l'air et à température ambiante. Après évaporation du solvant pendant 24 heures puis séchage sous vide pendant 6 heures, le film obtenu, constitué de polymère et de bicarbonate d'ammonium, a été décollé puis déposé dans de l'eau distillée chauffée à 70 °C. La présence d'eau à cette température entraîne une réaction avec le bicarbonate d'ammonium qui induit la formation d'ammoniac et de dioxyde de carbone. Les bulles de dioxyde de carbone en se formant et la disparition du sel donnent alors naissance à des pores dans la masse de polymère.

### 1.3. Synthèse d'un autre copolymère PLA2₅₀-PEG-PLA₅₀

Selon le même mode de synthèse, un autre copolymère a été synthétisé présentant une masse molaire moyenne en nombre de 310 000 g /mol.

Dans cet exemple, n = 2020 ; m = 2020 ; p = 454 et (n+m)/p = 8,9.

### Exemple 2: Etude de la dégradation hydrolytique de la matrice biocompatible

### 2.1 Matériel et méthode

### *Préparation du tampon de dégradation

Le milieu de dégradation retenu est un milieu de culture pour fibroblastes :
DMEM (Dulbecco's Modified Eagle's Medium) auquel sont ajoutés :
   - Sérum de veau nouveau-né (10%) (GIBCO-BRL), et
   - Pénicilline (100µg/ml), Streptomycine (100µg/ml) et amphotéricine B (1µg/ml) (GIBCO-BRL).

Pour suivre la dégradation du polymère, on a utilisé des films (L x 1 x H : 5 mm x 5 mm x 0,2 mm) obtenus par évaporation de solvant. Dans le cas de la dégradation des copolymères poreux, les pièces poreuses possédaient les mêmes dimensions que les films, exception faite de l'épaisseur (0,4 mm). Les pores ont été obtenus par la technique présentée ci-dessus. Les échantillons, de masses proches de 20-30 mg, ont été placés dans 1,5 ml de tampon de dégradation.

### Conditions de dégradation

- *In vitro* : les échantillons ont été placés dans un incubateur de culture cellulaire à 37 °C avec une atmosphère à 5 % en CO₂ saturée en eau.
- *In vivo* : les échantillons de matrice poreuse implantés dans le pli inguinal d'une souris ont subi après récupération un traitement enzymatique à la collagénase. Ce traitement en présence de chlorure de calcium (0,1 M) à 37 °C pendant 24 heures a permis de dissocier puis solubiliser les tissus ayant colonisé les matrices poreuses.

### 2.2 Dégradation comparative du PLA₅₀-PEG-PLA₅₀ et du PLA₅₀

Pour évaluer cette dégradation deux types d'étude ont été effectués :
- une étude *in vitro* pendant 24 semaines dans un incubateur de culture cellulaire.
- une étude *in vivo* pendant 19 semaines après implantation des supports chez des souris en sous cutanée.

### A. Etude de déaradation in vitro

Les objectifs de cette étude étaient les suivants :
- comparer la malléabilité du PLA₅₀-PEG-PLA₅₀ selon l'exemple 1.1. par rapport à celle d'un PLA₅₀ contrôle,
- évaluer la perte de masse du PLA₅₀ et du PLA₅₀-PEG-PLA₅₀ sur une période couvrant largement le temps nécessaire pour obtenir un substitut de tissu(s) de revêtement, et
- déterminer si la porosité a une influence sur la cinétique de dégradation.

Deux critères ont été retenus : l'aspect macroscopique et la perte de masse.

Pour le PLA₅₀ et le PLA₅₀-PEG-PLA₅₀, la dégradation de films (épaisseur de 200 µm) et de matrices poreuses (épaisseur de 400 µm) a été testée.

### a) Aspect microscopique et malléabilité

Avant la dégradation, tous les films et les matrices poreuses étaient semblables en couleur (blanche) et en souplesse. La couleur blanche est apparue lors de la stérilisation.

Après 3 semaines de dégradation, des différences d'aspect sont apparues très nettement. Les films de PLA₅₀ présentaient une surface bosselée et étaient enroulés sur eux-mêmes. Les matrices poreuses de PLA₅₀ ainsi que les films et les matrices poreuses de PLA₅₀-PEG-PLA₅₀ sont en revanche restées intactes. Concernant la souplesse, les échantillons de PLA₅₀-PEG-PLA₅₀ étaient toujours manipulables sans fragilité apparente.

Après 6 semaines de dégradation, les films et les matrices poreuses de PLA₅₀ avaient conservé leur aspect macroscopique, mais ils étaient devenus très délicats à manipuler car particulièrement friables. Par contre, les films et les matrices poreuses de PLA₅₀-PEG-PLA₅₀ avaient conservé leur aspect macroscopique sans apparition de surface ondulée ou bosselée et présentaient encore une grande souplesse compatible avec une manipulation chirurgicale.

Après 12 semaines de dégradation, la friabilité des échantillons de PLA₅₀ est apparue accentuée avec une impossibilité de récupérer les échantillons en un seul morceau. Les films de PLA₅₀-PEG-PLA₅₀ quant à eux, étaient enroulés sur eux-mêmes et avaient perdu de la souplesse et des propriétés mécaniques.

Après 24 semaines de dégradation, tous les échantillons se sont révélés impossibles à récupérer en un seul morceau. Aucun échantillon (film ou matrice) n'avait conservé des propriétés mécaniques suffisantes pour faire l'objet d'une manipulation chirurgicale.

### b) Perte de masse

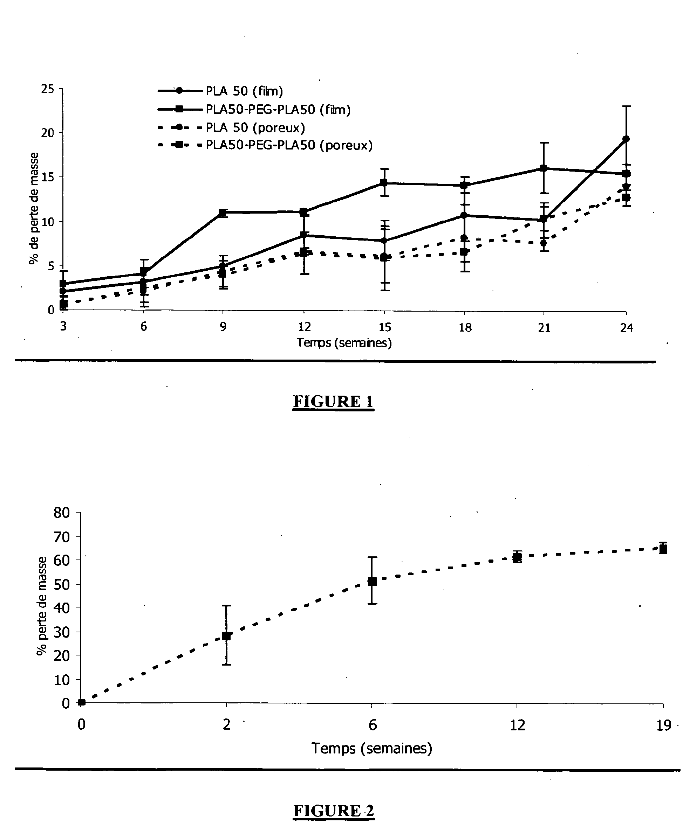

L'étude de dégradation du PLA₅₀ (films et matrices poreuses) et du PLA₅₀-PEG-PLA₅₀ (films et matrices poreuses) a été effectuée dans un incubateur de culture cellulaire, le milieu de dégradation étant le milieu de culture usuel pour fibroblastes. Les résultats sont rapportés à la Figure 1, jointe en annexe. Elle représente la perte de masse *in vitro* d'échantillons de PLA₅₀ (films et matrices poreuses) et de PLA₅₀-PEG-PLA₅₀ (films et matrices poreuses) sur une période de 24 semaines.

Les résultats correspondent à la moyenne de triplicate +/- l'écart type à la moyenne (SEM).

La Figure 1 montre que, après 6 mois, les diminutions de la masse des échantillons par rapport à la masse initiale sont de 19 % pour les films en PLA₅₀, 16 % pour les films en PLA₅₀-PEG-PLA₅₀, 14 % pour les matrices poreuses en PLA₅₀ et 13 % pour les matrices poreuses en PLA₅₀-PEG-PLA₅₀.

Cette étude de dégradation montre que les matrices poreuses en PLA₅₀-PEG-PLA₅₀ sont beaucoup plus malléables que les matrices poreuses en PLA₅₀. Leurs propriétés mécaniques sont conservées durant l'étude de dégradation *in vitro* pendant environ 12 semaines. La perte de masse pour les matrices poreuses n'excède pas 13 % après 24 semaines de dégradation. Le suivi de la perte de masse *in vitro* indique que les matrices poreuses se dégradent plus lentement que les films.

### B. Etude de dégradation in vivo

L'objectif de cette étude était d'évaluer la dégradation *in vivo* de la matrice poreuse en PLA₅₀-PEG-PLA₅₀ selon l'exemple 1.2. Pour cela, l'aspect macroscopique et la perte de masse des échantillons implantés en sous-cutané chez la souris ont été étudiés sur une période de 12 semaines.

### a) Aspect microscopique et malléabilité

Les échantillons implantés de matrice poreuse de PLA₅₀-PEG-PLA₅₀ avaient initialement une forme rectangulaire. L'évolution de la forme des échantillons implantés a permis d'observer partiellement la dégradation *in vivo.*

Les échantillons prélevés après 2 semaines d'implantation avaient conservé leur forme rectangulaire ainsi que leur malléabilité.

Après 6 semaines d'implantation, les échantillons présentaient des bords érodés et étaient toujours très malléables. Cette appréciation de la malléabilité a été cependant faussée par le fait que les matrices poreuses avaient été largement colonisées par les tissus environnants, ces tissus emprisonnés dans la matrice renforçant certainement les propriétés mécaniques de la matrice.

Après 12 et 19 semaines d'implantation, les échantillons ne possédaient plus leur forme rectangulaire initiale mais une forme ovale. Tout comme après 6 semaines d'implantation, il était difficile de juger de la malléabilité des matrices. Des délitements de la matrice étaient toutefois observables particulièrement pour les échantillons prélevés après 19 semaines.

### b) Perte de masse

La dégradation des échantillons implantés de matrices poreuses de PLA₅₀-PEG-PLA₅₀ a été étudiée par suivi de la perte de masse et représentée à la Figure 2, qui illustre la perte de masse *in vivo* d'échantillons de matrices poreuses de PLA₅₀-PEG-PLA₅₀ sur une période de 19 semaines. Les résultats correspondent à la moyenne de triplicate +/l'écart type à la moyenne (SEM). Pour peser uniquement la matrice poreuse et non les tissus qui l'ont colonisée, il a été nécessaire de traiter les échantillons avec la collagénase pour solubiliser les tissus.

D'après la Figure 2, les échantillons ont perdu 26 % de leur masse initiale après 2 semaines d'implantation, 52 % après 6 semaines, 62 % après 12 semaines et 66 % après 19 semaines.

L'étude de dégradation *in vivo* montre que les matrices poreuses se dégradent rapidement *in vivo,* la perte de masse étant de 66 % après 19 semaines. Ces différences de cinétique de dégradation observées lors des études *in vitro* et *in vivo* peuvent être expliquées par la présence des cellules qui par leurs migrations et leurs activités peuvent éroder la surface de la matrice. Il est également possible que la circulation permanente de fluides biologiques *in vivo* par rapport à la relative immobilité des milieux de dégradation *in vitro* contribue à stimuler la dégradation de la matrice poreuse, de même que la dynamique liée à la mobilité de l'animal.

### Exemple 3 : Cytocompatibilité cutanée et gingivale des matrices poreuses de PLA₅₀-PEG-PLA₅₀

Les kératinocytes sont isolés à partir de prépuces et cultivés dans un milieu sans sérum. Ce milieu permet la culture de kératinocytes en l'absence de couche nourricière (fibroblastes murins irradiés), l'extrait pituitaire bovin permet de remplacer le sérum foetal de veau. Ainsi, il est possible d'évaluer l'adhésion et la prolifération spécifique des kératinocytes.

Le milieu sans sérum est préparé à partir d'un volume de milieu MCDB153, auquel les suppléments suivants sont ajoutés :
- Extrait pituitaire bovin (70 µg/ml) (GIBCO-BRL),
- Facteur de croissance épidermique (EGF) (10 ng/ml) (GIBCO-BRL),
- Pénicilline (100 µg/ml), Streptomycine (100 µg/ml) et amphotéricine B (1 µg/ml) (GIBCO-BRL).

Les fibroblastes dermiques humains sont isolés à partir de prépuces. Les fibroblastes gingivaux humains sont isolés à partir d'une biopsie obtenue après avulsion de dent de sagesse. Les deux types de fibroblastes sont cultivés avec un milieu spécifique préparé extemporanément : DMEM (Dulbecco's Modified Eagle's Medium) auquel sont ajoutés :
- Sérum de veau nouveau-né (10%, vol.) (GIBCO-BRL),
- Pénicilline (100 µg/ml), Streptomycine (100 µg/ml) et amphotéricine B (1 µg/ml) (GIBCO-BRL).

La numération cellulaire est estimée à l'aide du test colorimétrique MTT. Le MTT (bromure de 3-(4,5-dimethyl thiazol-2-yl)-2,-5 diphenyl tetrazolium) est un substrat des succinates deshydrogénases qui sont des enzymes mitochondriales. Ces enzymes sont capables de transformer le MTT en cristaux de formazan insolubles en milieu aqueux. La solubilisation de ces cristaux dans l'isopropanol puis la mesure de la densité optique à 570 nm (SLT Spectra) permet d'évaluer l'activité mitochondriale et donc la viabilité cellulaire et ainsi proportionnellement le nombre de cellules.

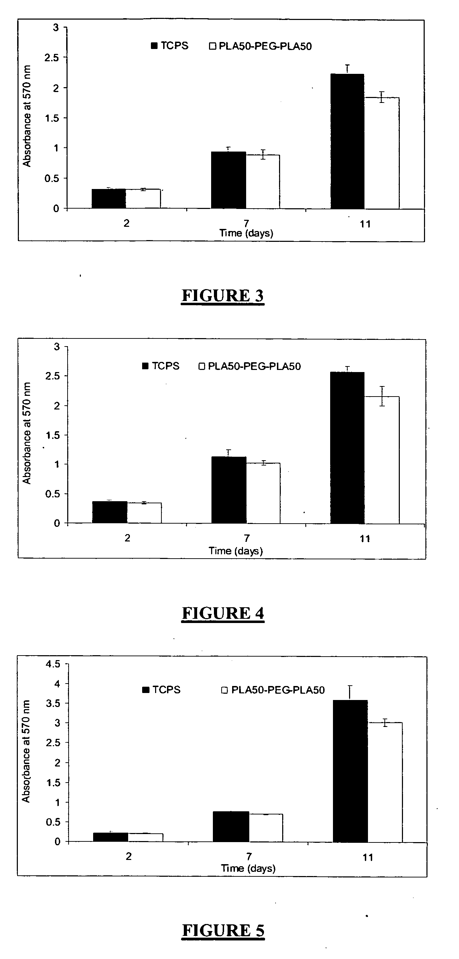

L'étude de la prolifération **fibroblastique** sur une période de 11 jours indique que les fibroblastes dermiques et gingivaux sont capables de proliférer sur les matrices poreuses en PLA₅₀-PEG-PLA₅₀ en comparaison avec le polystyrène (TCPS, tissue culture polystyrene) (voir Figures 3, 4).

La figure 3 illustre l'étude de la prolifération de fibroblastes dermiques sur des films de PLA₅₀-PEG-PLA₅₀ tandis que la figure 4 illustre l'étude de prolifération de fibroblastes gingivaux sur des films de PLA₅₀-PEG-PLA₅₀.

L'étude de la prolifération **kératinocytaire** sur une période de 11 jours indique que les kératinocytes sont capables de proliférer sur les supports en PLA₅₀-PEG-PLA₅₀ en comparaison avec le polystyrène (TCPS, tissue culture polystyrène) (Figure 5).

La figure 5 illustre l'étude de la prolifération de kératinocytes sur des film de PLA₅₀-PEG-PLA₅₀.

### Exemple 4: Utilisation de l'échafaudage de tissu(s) conjonctif(s) biocompatible en culture cellulaire

### 4.1. Pour la formation d'un substitut mésenchymateux

Le substitut mésenchymateux est composé d'une matrice poreuse en PLA₅₀-PEG-PLA₅₀ selon l'exemple 1.2. dont les pores sont chargés de collagène de type I de rat et des fibroblastes dermiques humains. Le collagène de type I est extrait à partir de tendons de queue de rat dissous dans une solution d'acide acétique à 0,1 % (vol./vol.). Après centrifugation, le collagène est dilué pour obtenir une solution à 3 mg/ml. A cette solution de collagène, sont ajoutés extemporanément et chronologiquement :
- DMEM/Sérum nouveau né 9/1 vol./vol. (milieu de culture pour fibroblastes dermiques humains),
- NaOH 0,1 N (50 µl/ml),
- Fibroblastes dermiques humains (300 000 cellules/ml).

Une fois le mélange effectué, celui-ci doit être immédiatement déposé sur la matrice poreuse avant que la réticulation des fibres de collagène dans les pores ne soit totale. La quantité finale de collagène dans le substitut mésenchymateux varie en masse de 0,05 à 7 % de la masse totale dudit substitut.

L'analyse histologique du substitut mésenchymateux révèle la présence de fibroblastes en contact avec l'échafaudage de tissu(s) conjonctif(s).

### 4.2 Pour la colonisation vasculaire

Le modèle d'angiogenèse *in vivo* utilisé consiste en l'implantation de matrices poreuses sans collagène dans le pli inguinal de souris C57/black-6 (Iffacredo) durant une période de 45 jours. La présence de collagène *in vitro* est obligatoire pour obtenir la formation de néovaisseaux, en revanche *in vivo,* le support seul peut être évalué. Trois aspects ont été particulièrement étudiés :
- la cytocompatibilité vasculaire ou « intimité » des néovaisseaux avec la matrice poreuse,
- la colonisation vasculaire ou capacité de l'organisme à vasculariser un implant poreux en PLA₅₀-PEG-PLA₅₀,
- la formation d'un réseau vasculaire.

Lors du retrait du tissu localisé à la surface de la matrice, des fragments de matrice poreuse sont restés attachés à ce tissu. Le fait que des fragments de matrice poreuse restent attachés au tissu néoformé rend compte de la proximité voire de l'intimité des néovaisseaux avec la surface de la matrice poreuse. En effet, certains vaisseaux entourent les fragments de matrice poreuse et des bourgeonnements vasculaires sont observables.
- L'injection intrajugulaire de 0,1 mg de lectine-AlexaFluor568 (isolectine 1B4, Bandeiraea simplicifolia, Molecular probes) dans 0,1 ml de sérum physiologique, permet la mise en évidence d'un réseau vasculaire organisé de manière hiérarchique.

La fonctionnalité du système vasculaire développé à l'intérieur de ce réseau 3D est démontrée par la présence de cellules musculaires lisses spécifiques des artères ou artérioles caractérisées par l'expression de l'α-SM actine (Monoclonal Anti alpha Smooth Muscle Actin, Mouse Ascites Fluid Clone 1A4, Sigma-Aldrich).

En conclusion, cette étude d'angiogenèse fournit les résultats suivants : 1/ la matrice poreuse est compatible avec les cellules d'origine vasculaire, il existe même une « intimité » des néovaisseaux avec la matrice poreuse, 2/ le système vasculaire est capable de coloniser un implant poreux en PLA₅₀-PEG-PLA₅₀. Un réseau vasculaire comprenant artérioles et veinules s'est organisé sur et dans la matrice poreuse.

### 4.3 Pour la reconstruction épidermique

Pour former un épiderme stratifié *in vitro,* une technique de culture en deux étapes a été utilisée, caractérisée par l'utilisation d'un milieu avec sérum. La première étape consiste à co-cultiver en milieu immergé des kératinocytes et des fibroblastes murins (Technique développée par Jim Rheinwald et Howard Green).

Ces fibroblastes murins (J2) dont la prolifération est inhibée par un traitement à la mitomycine C (4 µg/ml) pendant 3 heures, jouent le rôle de cellules nourricières en sécrétant des protéines de la matrice extra-cellulaire qui favorisent l'attachement des kératinocytes et des facteurs de croissance qui stimulent la prolifération des kératinocytes.

Après avoir obtenu un feuillet monocellulaire de kératinocytes, la deuxième étape consiste à placer le support de culture de kératinocytes sur une grille qui permet à l'épiderme de se situer à l'interface air-liquide. La mise à l'interface air-liquide permet de mimer la situation physiologique de l'épiderme et ainsi favoriser la stratification épidermique.

La composition du milieu de culture avec sérum pour les deux étapes précédemment décrites est la suivante :
- Milieu DMEM/HAM F12 (GIBCO-BRL) (2/1 : vol./vol.)
   - Sérum de veau foetal (10% vol./vol.) (GIBCO-BRL),
   - Hydrocortisone (0,4 mg/ml) (SIGMA-ALDRICH),
   - Insuline (5 mg/ml) (SIGMA-ALDRICH),
   - Pénicilline (100 µg/ml), Streptomycine (100 µg/ml) et amphotéricine B (1 µg/ml) (GIBCO-BRL),
   - Facteur de croissance épidermique (EGF) (10 ng/ml) (SIGMA-ALDRICH), et
   - Toxine cholérique (0,1 nM) (SIGMA-ALDRICH).

Après 20 jours de culture, les échantillons ont été coupés à l'aide d'un cryomicrotome. Les coupes de 5 à 15 µm sont fixées avec du méthanol froid et une solution de paraformaldéhyde à 4%. Les marquages par immunofluorescence indirecte ont été obtenus avec des anticorps de souris spécifiques de la kératine 10, la kératine 6, la kératine 14, l'involucrine, la E-cadhérine et l'intégrine β1. L'anticorps secondaire utilisé est un anticorps anti-souris obtenus chez la chèvre couplé à un dérivé de la fluorescéine (FITC : Fluoresceine Iso Thio Cyanate). L'ADN nucléaire a été marqué à l'aide d'un colorant: le Hoechst 33342 (SIGMA-ALDR7CH).

L'analyse histologique du substitut de tissu(s) de revêtement, révèle la présence d'un épiderme comportant plusieurs couches cellulaires cultivé sur le support en matrice poreuse ainsi que la présence de fibroblastes dans le compartiment dermique. Le marquage de l'ADN permet d'apprécier la morphologie des kératinocytes. Nous observons que la plupart des cellules des couches supérieures de l'épiderme formées sur la matrice poreuse comportent un noyau fusiforme et aplati. Cette morphologie est caractéristique des kératinocytes composant la couche granuleuse. Ces observations prouvent que les kératinocytes, après avoir colonisé la surface de la matrice poreuse se sont engagés dans le programme de différentiation terminale formant ainsi un épiderme stratifié après 20 jours de culture.

La caractérisation de cet épiderme par immunohistologie permet de conclure à la présence des différents marqueurs spécifiques de l'épiderme.

### Exemples 5 : Intégration in vivo du substitut de tissu(s) de revêtement

Après avoir fabriqué un substitut cutané comportant un équivalent dermique composé d'une matrice poreuse en PLA₅₀-PEG-PLA₅₀ telle que synthétisée à l'exemple 1.3, de collagène de rat de type 1 et de fibroblastes dermiques humains, et un épiderme humain pluristratifié jouant le rôle de barrière, il est apparu essentiel d'évaluer la possibilité d'intégrer ce substitut de tissu(s) de revêtement in vivo.

Dans cette optique, des échantillons de ce type de substitut de tissu(s) de revêtement ont été greffés sur le dos de souris Nude (souris immunodéprimé permettant d'éviter le rejet des cellules humaines) après avoir prélevé un fragment de peau de souris de dimensions correspondantes au substitut cutané (L x 1 : 7 mm x 7 mm). La greffe et la cicatrisation d'un substitut de tissu(s) de revêtement ont été suivies durant une période de 27 jours. Nous observons tout d'abord que 4 jours après la greffe, le contour du greffon est enflammé et que la surface épidermique du substitut conserve la même coloration qu'avant la greffe. 27 jours après la greffe, un réseau vasculaire s'est organisé à la périphérie du substitut et semble le coloniser. Ce substitut apparaît donc comme un bon support de migration pour les kératinocytes murins des berges de la plaie. Cette colonisation indique que la souris n'a pas rejeté le substitut de tissu(s) de revêtement.

Tous les exemples démontrent que la matrice poreuse réalisée de PLA₅₀-PEG-PLA₅₀ est apte à former un substitut mésenchymateux pour une utilisation à titre de substitut de tissu(s) de revêtement et/ou à titre de pansement bioactif.

## Revendications

1. Matrice poreuse biocompatible et biodégradable, **caractérisée en ce qu'**elle comprend un copolymère séquencé tribloc répondant à la formule (I) :
X -G -Y (I)
dans laquelle :
G est un bloc linéaire hydrophile, non hydroxylé, contenant p motifs répétitifs, p étant un nombre pouvant varier de 150 à 700,
X et Y, identiques ou différents, représentent respectivement un bloc polyester linéaire hydrophobe, X et Y pouvant chacun respectivement contenir n et m motifs répétitifs, n et m étant chacun un nombre pouvant varier de 170 à 3500,
et **en ce qu'**elle comprend des pores de diamètre variant de 20 à 500 µm.

2. Matrice selon la revendication 1, **caractérisée en ce que** le diamètre desdits pores varie de 150 à 500 µm.

3. Matrice selon la revendication 1 ou 2, **caractérisée en ce que** n et m varient indépendamment l'un de l'autre de 200 à 3000, et notamment de 300 à 2500.

4. Matrice selon l'une quelconque des revendications 1 à 3, **caractérisée en ce que** p varie de 180 à 650, et notamment de 200 à 600.

5. Matrice selon l'une quelconque des revendications 1 à 4, **caractérisée en ce que** le rapport (m+n))/p est compris entre 0,48 et 47.

6. Matrice selon l'une quelconque des revendications 1 à 5, **caractérisée en ce que** le rapport (m+n))/p est compris entre 0,60 et 33, et notamment entre 1 et 25.

7. Matrice selon l'une quelconque des revendications 1 à 6, **caractérisée en ce qu'**elle présente une masse molaire moyenne en nombre comprise entre 65 000 et 400 000 et présente un rapport (m+n)/p comprise entre 1 et 15.

8. Matrice selon l'une quelconque des revendications 1 à 7, 30 **caractérisée en ce qu'**elle présente une masse molaire moyenne en nombre comprise entre 80 000 et 375 000 et présente un rapport (m+n)/p comprise entre 1,8 et 10,9.

9. Matrice selon l'une quelconque des revendications 1 à 8, **caractérisée en ce que** X et Y identiques ou différents représentent respectivement (i) un homopolymere de monomères choisis parmi l'acide lactique, les monoesters d'acide malique, les lactides, le glycolide, la para-dioxanone, l'ε-caprolactone, (ii) un copolymère desdits monomères, ou (iii) un polymère obtenu par polycondensation de diacides et de diols.

10. Matrice selon l'une quelconque des revendications 1 à 9, **caractérisée en ce que** X et Y identiques ou différents représentent respectivement un homopolymère de monomères choisis parmi l'acide lactique, les monoesters d'acide malique, les lactides, le glycolide, la para-dioxanone, l'ε-caprolactone.

11. Matrice selon l'une quelconque des revendications 1 à 10, **caractérisée en ce que** le bloc G est choisi parmi un poly(éthylène glycol), un poly(vinylpyrrolidone),poly(vinyl alcohol), polyoxazoline.

12. Matrice selon l'une quelconque des revendications 1 à 11, **caractérisée en ce que** le bloc G est un bloc poly(éthylène glycol) de formule H(OCH₂CH₂)_{P}OH où p varie de 200 à 600.

13. Matrice selon l'une quelconque des revendications 1 à 12, **caractérisée en ce que** X et Y sont identiques et sont chacun un homopolymère de D,L-lactides, **en ce que** G est un poly(éthylène glycol) à 20 000 g/mol, et **en ce que**
m = n = 451 p = 454 et (m+n)/p = 1,98 ; ou
m = n = 2020 p = 454 et (m+n)/p = 8,9.

14. Procédé de préparation d'une matrice selon l'une quelconque des revendications 1 à 13, **caractérisé en ce qu'**il comprend au moins une étape de préparation d'un copolymère tel que défini dans l'une quelconque des revendications 1 à 13, suivie d'une étape de formation de pores au sein du copolymère ainsi préparé.

15. Procédé selon la revendication 14, **caractérisé en ce que** l'étape de formation de pores met en oeuvre, à titre d'agent générateur de porosité, des particules comprenant de l'hydrogénocarbonate d'ammonium.

16. Echafaudage de tissu(s) conjonctif(s) **caractérisé en ce qu'**il comprend au moins une matrice selon l'une quelconque des revendications 1 à 13 et au moins un agent de comblement biocompatible et dégradable.

17. Echafaudage selon la revendication précédente, **caractérisé en ce que** l'agent de comblement est choisi parmi un matériau, biologique ou non, contenant du collagène et/ou de la fibrine.

18. Echafaudage selon la revendication 17, **caractérisé en ce que** l'agent de comblement est du plasma riche en fibrine coagulée.

19. Echafaudage selon la revendication 17, **caractérisé en ce que** l'agent de comblement est un gel de collagène.

20. Substitut mésenchymateux utile pour la reconstruction de tissu(s) conjonctif(s) et/ou pour la cicatrisation, **caractérisé en ce qu'**il comprend une matrice poreuse selon l'une quelconque des revendications 1 à 13 ou un échafaudage de tissu(s) conjonctif(s) selon l'une quelconque des revendications 16 à 19, en association avec des cellules endothéliales et/ou fibroblastes.

21. Procédé de préparation du substitut mésenchymateux selon la revendication 20, **caractérisé en ce qu'**il comprend une étape de mise en contact d'une matrice poreuse selon l'une quelconque des revendications 1 à 13 ou d'un échafaudage de tissu(s) conjonctif(s) selon l'une quelconque des revendications 16 à 19 avec des cellules endothéliales et/ou fibroblastes, puis une étape de prolifération cellulaire.

22. Substitut de tissu(s) de revêtement, **caractérisé en ce qu'**il comprend au moins une matrice poreuse selon l'une quelconque des revendications 1 à 13, un échafaudage de tissu(s) conjonctif(s) selon l'une quelconque des revendications 16 à 19, ou un substitut mésenchymateux selon la revendication 20, en association avec des cellules épithéliales.

23. Procédé de préparation du substitut de tissu(s) de revêtement selon la revendication 22, **caractérisé en ce qu'**il comprend une étape de mise en contact d'une matrice poreuse selon l'une quelconque des revendications 1 à 13, d'un échafaudage de tissu(s) conjonctif(s) selon l'une quelconque des revendications 16 à 19 et/ou d'un substitut mésenchymateux selon la revendication 20 avec des cellules épithéliales, puis une étape de prolifération cellulaire.

24. Utilisation de la matrice poreuse selon l'une quelconque des revendications 1 à 13, pour l'élaboration d'un échafaudage de tissu(s) conjonctif(s) selon l'une quelconque des revendications 16 à 19.

25. Utilisation d'un échafaudage de tissu(s) conjonctif(s) selon l'une quelconque des revendications 16 à 19, pour l'obtention d'un substitut mésenchymateux selon la revendication 20.

26. Utilisation d'un substitut mésenchymateux selon la revendication 20, pour l'obtention d'un substitut de tissu(s) de revêtement selon la revendication 22.

27. Utilisation d'une matrice poreuse selon l'une quelconque des revendications 1 à 13, d'un échafaudage de tissu(s) conjonctif(s) selon l'une quelconque des revendications 16 à 19, d'un substitut mésenchymateux selon la revendication 20 ou d'un substitut de tissu(s) de revêtement selon la revendication 22, pour la préparation d'un matériau destiné à la réparation de téguments et/ou pour la préparation d'un pansement bioactif destiné à favoriser la cicatrisation.

28. Utilisation d'une matrice poreuse selon l'une quelconque des revendications 1 à 13, d'un échafaudage de tissu(s) conjonctif(s) selon l'une quelconque des revendications 16 à 19, d'un substitut mésenchymateux selon la revendication 20 ou d'un substitut de tissu(s) de revêtement selon la revendication 22 à des fins de réalisation d'un test de diagnostic *in vitro.*

## Patentansprüche

1. Poröse biokompatible und biologisch abbaubare Matrix, **dadurch gekennzeichnet, dass** sie ein Triblockcopolymer entsprechend der Formel (I) umfasst:
X - G - Y (I)
in der:
G ein linearer hydrophiler, nicht hydroxylierter, Block ist, der p repetitive Motive enthält, wobei p eine Zahl ist, die von 150 bis 700 variieren kann,
X und Y, identisch oder verschieden, jeweils einen linearen hydrophoben Polyesterblock darstellen, wobei jedes von X und Y jeweils n und m repetitive Motive enthalten kann, wobei jedes von n und m eine Zahl ist, die von 170 bis 3500 variieren kann,
und dadurch, dass sie Poren mit einem von 20 bis 500 µm variierenden Durchmesser umfasst.

2. Matrix nach Anspruch 1, **dadurch gekennzeichnet, dass** der Durchmesser der Poren von 150 bis 500 µm variiert.

3. Matrix nach Anspruch 1 oder 2, **dadurch gekennzeichnet, dass** n und m unabhängig voneinander von 200 bis 3000, und insbesondere von 300 bis 2500 variieren.

4. Matrix nach einem beliebigen der Ansprüche 1 bis 3, **dadurch gekennzeichnet, dass** p von 180 bis 650, und insbesondere von 200 bis 600 variiert.

5. Matrix nach einem beliebigen der Ansprüche 1 bis 4, **dadurch gekennzeichnet, dass** das Verhältnis (m+n)/p zwischen 0,48 und 47 liegt.

6. Matrix nach einem beliebigen der Ansprüche 1 bis 5, **dadurch gekennzeichnet, dass** das Verhältnis (m+n)/p zwischen 0,60 und 33, und insbesondere zwischen 1 und 25 liegt.

7. Matrix nach einem beliebigen der Ansprüche 1 bis 6, **dadurch gekennzeichnet, dass** sie eine zahlengemittelte molare Masse aufweist, die zwischen 65 000 und 400 000 liegt, und ein Verhältnis (m+n)/p aufweist, das zwischen 1 und 15 liegt.

8. Matrix nach einem beliebigen der Ansprüche 1 bis 7, **dadurch gekennzeichnet, dass** sie eine zahlengemittelte molare Masse aufweist, die zwischen 80 000 und 375 000 liegt, und ein Verhältnis (m+n)/p aufweist, das zwischen 1,8 und 10,9 liegt.

9. Matrix nach einem beliebigen der Ansprüche 1 bis 8, **dadurch gekennzeichnet, dass** X und Y, identisch oder verschieden, jeweils (i) ein Homopolymer aus Monomeren ausgewählt aus Milchsäure, Monoestern von Äpfelsäure, Lactiden, Glycolid, para-Dioxanon, ε-Caprolacton, (ii) ein Copolymer dieser Monomere, oder (iii) ein Polymer, das durch Polykondensation von Disäuren und Diolen erhalten ist, darstellen.

10. Matrix nach einem beliebigen der Ansprüche 1 bis 9, **dadurch gekennzeichnet, dass** X und Y, identisch oder verschieden, jeweils ein Homopolymer aus Monomeren ausgewählt aus Milchsäure, Monoestern von Äpfelsäure, Lactiden, Glycolid, para-Dioxanon, ε-Caprolacton darstellen.

11. Matrix nach einem beliebigen der Ansprüche 1 bis 10, **dadurch gekennzeichnet, dass** Block G ausgewählt ist aus einem Poly(ethylenglycol), einem Poly(vinylpyrrolidon), Poly(vinylalkohol), Polyoxazolin.

12. Matrix nach einem beliebigen der Ansprüche 1 bis 11, **dadurch gekennzeichnet, dass** Block G ein Block-Poly(ethylenglykol) der Formel H(OCH₂CH₂)ₚOH ist, wo p von 200 bis 600 variiert.

13. Matrix nach einem beliebigen der Ansprüche 1 bis 12, **dadurch gekennzeichnet, dass** X und Y identisch sind, und jedes ein Homopolymer aus D,L-Lactiden ist, dadurch, dass G ein Poly(ethylenglykol) mit 20 000 g/mol ist, und dadurch, dass
m = n = 451 p = 454 und (m+n)/p = 1,98; oder
m = n = 2020 p = 454 und (m+n)/p = 8,9.

14. Verfahren zur Herstellung einer Matrix gemäß einem beliebigen der Ansprüche 1 bis 13, **dadurch gekennzeichnet, dass** es mindestens einen Schritt der Herstellung eines Copolymers wie in einem beliebigen der Ansprüche 1 bis 13 definiert gefolgt von einem Schritt des Bildens von Poren innerhalb des so hergestellten Copolymers umfasst.

15. Verfahren nach Anspruch 14, **dadurch gekennzeichnet, dass** der Schritt des Bildens von Poren als Porositätserzeugungsmittel Partikel, die Ammoniumhydrogencarbonat umfassen, einsetzt.

16. Bindegewebegerüst, **dadurch gekennzeichnet, dass** es mindestens eine Matrix gemäß einem beliebigen der Ansprüche 1 bis 13 und mindestens einen biokompatiblen und abbaubaren Füllstoff umfasst.

17. Gerüst nach dem voranstehenden Anspruch, **dadurch gekennzeichnet, dass** der Füllstoff ausgewählt ist aus einem Material, biologisch oder nicht, das Kollagen und/oder Fibrin enthält.

18. Gerüst nach Anspruch 17, **dadurch gekennzeichnet, dass** der Füllstoff Plasma ist, das reich an koaguliertem Fibrin ist.

19. Gerüst nach Anspruch 17, **dadurch gekennzeichnet, dass** der Füllstoff ein Kollagengel ist.

20. Mesenchymaler Ersatz, nützlich zur Rekonstruktion von Bindegewebe(n) und/oder zur Wundheilung, **dadurch gekennzeichnet, dass** er eine poröse Matrix gemäß einem beliebigen der Ansprüche 1 bis 13 oder ein Bindegewebegerüst gemäß einem beliebigen der Ansprüche 16 bis 19, in Assoziation mit Endothelzellen und/oder Fibroblasten, umfasst.

21. Verfahren zur Herstellung des mesenchymalen Ersatzes gemäß Anspruch 20, **dadurch gekennzeichnet, dass** es einen Schritt des Inkontaktbringens einer porösen Matrix gemäß einem beliebigen der Ansprüche 1 bis 13 oder eines Bindegewebegerüsts nach einem beliebigen der Ansprüche 16 bis 19 mit Endothelzellen und/oder Fibroblasten, anschließend einen Schritt der Zellproliferation, umfasst.

22. Deckgewebeersatz, **dadurch gekennzeichnet, dass** er mindestens eine poröse Matrix gemäß einem beliebigen der Ansprüche 1 bis 13, ein Bindegewebegerüst gemäß einem beliebigen der Ansprüche 16 bis 19, oder einen mesenchymalen Ersatz gemäß Anspruch 20, in Assoziation mit Epithelzellen, umfasst.

23. Verfahren zur Herstellung des Deckgewebeersatzes gemäß Anspruch 22, **dadurch gekennzeichnet, dass** es einen Schritt des Inkontaktbringens einer porösen Matrix gemäß einem beliebigen der Ansprüche 1 bis 13, eines Bindegewebegerüsts gemäß einem beliebigen der Ansprüche 16 bis 19, und/oder eines mesenchymalen Ersatzes gemäß Anspruch 20 mit Epithelzellen, anschließend einen Schritt der Zellproliferation, umfasst.

24. Verwendung der porösen Matrix gemäß einem beliebigen der Ansprüche 1 bis 13 zum Erstellen eines Bindegewebegerüsts gemäß einem beliebigen der Ansprüche 16 bis 19.

25. Verwendung eines Bindegewebegerüsts gemäß einem beliebigen der Ansprüche 16 bis 19 zum Erhalten eines mesenchymalen Ersatzes gemäß Anspruch 20.

26. Verwendung eines mesenchymalen Ersatzes gemäß Anspruch 20 zum Erhalten eines Deckgewebeersatzes gemäß Anspruch 22.

27. Verwendung einer porösen Matrix gemäß einem beliebigen der Ansprüche 1 bis 13, eines Bindegewebegerüsts gemäß einem beliebigen der Ansprüche 16 bis 19, eines mesenchymalen Ersatzes gemäß Anspruch 20 oder eines Deckgewebeersatzes gemäß Anspruch 22 zur Herstellung eines Materials, das zur Reparatur von Integumenten bestimmt ist, und/oder zur Herstellung eines bioaktiven Verbandsmaterials, das zum Fördern der Wundheilung bestimmt ist.

28. Verwendung einer porösen Matrix gemäß einem beliebigen der Ansprüche 1 bis 13, eines Bindegewebegerüsts gemäß einem beliebigen der Ansprüche 16 bis 19, eines mesenchymalen Ersatzes gemäß Anspruch 20 oder eines Deckgewebeersatzes gemäß Anspruch 22 zu Zwecken der Realisierung eines diagnostischen Tests *in vitro.*

## Claims

1. A biocompatible and biodegradable porous matrix, **characterized in that** it comprises a three-block sequenced copolymer which satisfies the formula (I):
X-G-Y (I)
in which:
G is a hydrophilic linear non-hydroxylated block containing p repeating units, with p being a number which may vary from 150 to 700,
X and Y, which may be identical or different, respectively represent a hydrophobic linear polyester block, X and Y each possibly containing n and m repeating units respectively, n and m each being a number which may vary from 170 to 3500,
and **in that** it comprises pores with a diameter varying from 20 to 500 µm.

2. The matrix as claimed in claim 1, **characterized in that** the diameter of said pores varies from 150 to 500 µm.

3. The matrix as claimed in claim 1 or claim 2, **characterized in that** n and m vary independently of each other from 200 to 3000, and in particular from 300 to 2500.

4. The matrix as claimed in any one of claims 1 to 3, **characterized in that** p varies from 180 to 650, and in particular from 200 to 600.

5. The matrix as claimed in any one of claims 1 to 4, **characterized in that** the ratio (m+n)/p is in the range 0.48 to 47.

6. The matrix as claimed in any one of claims 1 to 5, **characterized in that** the ratio (m+n)/p is in the range 0.60 to 33, and in particular in the range 1 to 25.

7. The matrix as claimed in any one of claims 1 to 6, **characterized in that** it has a number average molar mass in the range 65000 to 400000 and has a ratio (m+n)/p in the range 1 to 15.

8. The matrix as claimed in any one of claims 1 to 7, **characterized in that** it has a number average molar mass in the range 80000 to 375000 and has a ratio (m+n)/p in the range 1.8 to 10.9.

9. The matrix as claimed in any one of claims 1 to 8, **characterized in that** X and Y, which may be identical or different, respectively represent (i) a homopolymer produced from monomers selected from lactic acid, monoesters of malic acid, lactides, glycolide, para-dioxanone, and ε-caprolactone, (ii) a copolymer of said monomers, or (iii) a polymer obtained by polycondensation of diacids and diols.

10. The matrix as claimed in any one of claims 1 to 9, **characterized in that** X and Y, which may be identical or different, respectively represent a homopolymer produced from monomers selected from lactic acid, monoesters of malic acid, lactides, glycolide, para-dioxanone and ε-caprolactone.

11. The matrix as claimed in any one of claims 1 to 10, **characterized in that** the block G is selected from the group consisting of a poly(ethylene glycol), a poly(vinylpyrrolidone), poly(vinyl alcohol) and polyoxazoline.

12. The matrix as claimed in any one of claims 1 to 11, **characterized in that** the block G is a poly(ethylene glycol) block with formula H(OCH₂CH₂)ₚOH, in which p varies from 200 to 600.

13. The matrix as claimed in any one of claims 1 to 12, **characterized in that** X and Y are identical and are each a homopolymer of D,L-lactides, **in that** G is a 20000 g/mol poly(ethylene glycol), and **in that**
m = n = 451 p = 454 and (m+n)/p = 1.98; or
m = n = 2020 p = 454 and (m+n)/p = 8.9.

14. A method for the preparation of a matrix as claimed in any one of claims 1 to 13, **characterized in that** it comprises at least one step for preparing a copolymer as defined in any one of claims 1 to 13, followed by a step for forming pores within the prepared copolymer.

15. The method as claimed in claim 14, **characterized in that** the pore formation step employs particles comprising ammonium hydrogen carbonate as a pore-forming agent.

16. A scaffold of conjunctive tissue(s), **characterized in that** it comprises at least one matrix as claimed in any one of claims 1 to 13 and at least one biocompatible and degradable filling agent.

17. The scaffold as claimed in the preceding claim, **characterized in that** the filling agent is selected from a material which may or may not be biological and which contains collagen and/or fibrin.

18. The scaffold as claimed in claim 17, **characterized in that** the filling agent is plasma which is rich in coagulated fibrin.

19. The scaffold as claimed in claim 17, **characterized in that** the filling agent is a collagen gel.

20. A mesenchymal substitute for the reconstruction of conjunctive tissue(s) and/or for healing, **characterized in that** it comprises a porous matrix as claimed in any one of claims 1 to 13 or a scaffold of conjunctive tissue(s) as claimed in any one of claims 16 to 19, in association with endothelial cells and/or fibroblasts.

21. A method for preparing the mesenchymal substitute as claimed in claim 20, **characterized in that** it comprises a step for placing a porous matrix as claimed in any one of claims 1 to 13 or a scaffold of conjunctive tissue(s) as claimed in any one of claims 16 to 19 into contact with endothelial cells and/or fibroblasts, followed by a cell proliferation step.

22. A coating tissue substitute, **characterized in that** it comprises at least one porous matrix as claimed in any one of claims 1 to 13, a scaffold of conjunctive tissue(s) as claimed in any one of claims 16 to 19, or a mesenchymal substitute as claimed in claim 20, in association with epithelial cells.

23. A method for preparing a coating tissue substitute as claimed in claim 22, **characterized in that** it comprises a step for bringing a porous matrix as claimed in any one of claims 1 to 13, a scaffolding of conjunctive tissue as claimed in any one of claims 16 to 19 and/or a mesenchymal substitute as claimed in claim 20 into contact with epithelial cells, followed by a step for cellular proliferation.

24. Use of the porous matrix as claimed in any one of claims 1 to 13, for the production of a scaffolding of conjunctive tissue(s) as claimed in any one of claims 16 to 19.

25. Use of a scaffolding of conjunctive tissue(s) as claimed in any one of claims 16 to 19, for the production of a mesenchymal substitute as claimed in claim 20.

26. Use of a mesenchymal substitute as claimed in claim 20, for the production of a coating tissue(s) substitute as claimed in claim 22.

27. Use of a porous matrix as claimed in any one of claims 1 to 13, of a scaffolding of conjunctive tissue(s) as claimed in any one of claims 16 to 19, of a mesenchymal substitute as claimed in claim 20 or of a coating tissue(s) substitute as claimed in claim 22, for the preparation of a material for repairing integuments and/or for the preparation of a bioactive dressing to promote healing.

28. Use of a porous matrix as claimed in any one of claims 1 to 13, of a scaffolding of conjunctive tissue(s) as claimed in any one of claims 16 to 19, of a mesenchymal substitute as claimed in claim 20 or of a coating tissue(s) substitute as claimed in claim 22, for the purposes of carrying out an *in vitro* diagnostic test.
